# EUROPEAN PATENT SPECIFICATION

(11) **EP 1 799 239 B1**
(45) Date of publication and mention of the grant of the patent: **07.05.2008**
(21) Application number: 05805094.9
(22) Date of filing: 21.09.2005
(51) Int. Cl.: A61K 36/82, A61K 127/00, A61K 135/00, A23F 3/16

(54) **PROCESS FOR MAKING TEA EXTRACTS**
VERFAHREN ZUR ZUBEREITUNG VON TEEEXTRAKTEN
PROCEDE DE PREPARATION D'EXTRAITS DE THE

(30) Priority: 01.10.2004 GB 0421827
(43) Date of publication of application: 27.06.2007
(73) Proprietor: UNILEVER PLC, London, Greater London EC4P 4BQ (GB); UNILEVER N.V., 3013 AL Rotterdam (NL)
(72) Inventor: MAVROUDIS, Nikolaos, Unilever R&D Vlaardingen, NL-3133 AT Vlaardingen (NL)
(74) Representative: Hugot, Alain
(86) International application number: PCT/EP2005/010376
(87) International publication number: WO 2006/037503

(56) References cited:
- EP-A- 0 267 660
- WO-A-95/18540
- DE-A1- 10 106 216
- US-A- 6 063 428
- ZHU XIAOLAN ET AL: "Simultaneous analysis of theanine, chlorogenic acid, purine alkaloids and catechins in tea samples with the help of multi-dimension information of on-line high performance liquid chromatography/electrospray-mass spectrometry." JOURNAL OF PHARMACEUTICAL AND BIOMEDICAL ANALYSIS. 18 FEB 2004, vol. 34, no. 3, 18 February 2004 (2004-02-18), pages 695-704, XP002366643 ISSN: 0731-7085

## Description

The present invention relates to a process for making theanine-rich tea extract.

### BACKGROUND AND PRIOR ART

Tea is generally prepared as green leaf tea or black leaf tea. The method of preparing such teas is well known to those skilled in the art. Generally, to prepare black leaf tea, fresh green leaves of the plant *Camellia sinensis* are withered (subjected to mild drying), comminuted, fermented (in which enzymes in the leaf tea oxidise various substrates to produce brown-coloured products) and then fired (to dry the tea leaves). Green leaf tea is not exposed to the fermentation process. Partial fermentation may be used to produce intermediate-type teas known as "oolong" tea.

Today tea based beverages can be prepared by methods other than infusing leaves in hot water and served in ways other than poured from tea pots. For example they can be made with concentrates or powders that are mixed with hot water in vending machines or used to prepare ready to drink teas in cans and bottles. Consumers also demand more from tea such as accelerated infusion, more colour, more aroma.

In particular the modern consumer is particularly interested in naturally healthy beverages which form part of a modern healthy lifestyle. As a beverage, tea fits well with this attitude in view of its natural content of inter alia flavonoids, catechins and amino acids. There is therefore a need in the art to provide a method for the concentration of these naturally occurring healthy ingredients whilst maintaining the healthy nature of tea without adding synthetic compound

One such ingredient is theanine. Theanine has been found to have numerous beneficial effects on the human body and mind. However, currently this is only available in high quantities in a synthetic sun-theanine form. This is largely due to the fact that naturally occurring theanine only comprises about 1% of the extractable tea solids in tea plant material.

GB 559,758 discloses a cold water infusion followed by a hot water infusion of black tea leaves. The cold water extract and the hot water extract are separately dried to a powder. The cold water infusion step takes at least 4 hours.

EP 110 391 discloses a cold water infusion followed by a hot water infusion of black leaf tea in order to provide an instant cold water-soluble ice tea powder. The cold water infusion step is shorter than in GB 559,758, and is exemplified by an extraction at room temperature for 10 minutes. The two extracts are mixed together, the mixture is then concentrated and then dried.

EP 267 660 again discloses a cold water infusion followed by a hot water infusion of black leaf tea but in order to provide a hot-water instant tea powder.

Yverong et al., "Effect of extraction temperature on cream and extractability of black tea" Int. Journal of Food Sci and Tech (2003), 38, 37-45 discloses that a water extraction of as high as 50°C results in the majority of the components responsible for formation of the cream remain unextracted. However, because an extraction at 50°C gives a low yield, leaving a large amount of tea solids in the leaf, the paper suggests that a second infusion at 90°C could follow so that the remaining tea solids can be extracted for normal hot drinking instant tea purposes.

WO2005/042470 discloses a process for extracting theanine from tea comprising the steps of extraction, contact with an adsorbent and then filtration. This does not disclose a short cold extraction and the preferred initial extraction involves steeping tea leaves in hot water.

The present inventors have discovered that performing a short cold water extraction on fermented tea leaves, provides a very effective way of extracting a high percentage of theanine from the tea leaves whilst leaving behind the vast majority of the remaining tea solids. However the composition of the tea solids in a cold-water extract are naturally limited to relative solubility's of the tea solids in the tea plant and are unlikely to comprise more than 6 wt% theanine. Therefore there remains a need in the art for a method of providing an even higher naturally occurring theanine concentration tea extract.

Thus, the present invention provides a process to provide a theanine-rich tea extract comprising the steps of:
(i) performing a cold water extraction of tea plant material using water at a temperature of from 1 to 30°C for a time period of from 1 to 120 minutes to provide a cold-water tea extract;
(ii) passing the extract through a microfiltration and a nanofiltration step wherein the nanofilter has a theanine rejection of less than 50% to provide a permeate having tea solids comprising from 8 to 40 wt% theanine.

In a second aspect, the invention provides a cold-water tea extract comprising tea solids, characterised in that the tea solids comprise from 8 to 40 wt% naturally occurring theanine.

### DETAILED DESCRIPTION OF THE INVENTION

### Tea starting material

The starting material of the present invention is tea plant material. Material from *Camellia sinensis, Camellia assamica*, or *Aspalathus linearis.* Preferably the starting material is black tea, in which the leaves and/or stem are subjected to a so-called "fermentation" step wherein they are oxidised by certain endogenous enzymes that are released during the early stages of "black tea" manufacture. This oxidation may even be supplemented by the action of exogenous enzymes such as oxidases, laccases and peroxidases. The fermentation process is believed to polymerise the polyphenols which may cause difficulties with the.sensitive filters used in the present invention.

### Cold water extraction

The cold water extraction is carried out with water at a temperature of from 1 to 30°C for a time period of from 1 to 120 minutes. Preferably, the temperature and duration are such that the product of the temperature in degrees Celsius and the duration of the extraction in minutes (Cmins) is from 30 to 1000, preferably from 100 to 500.

The cold water extraction step may be carried out in a batchwise or continuous manner. When run continuously, the extraction time refers to the mean residence time of the tea leaf.

Preferably the water is at a temperature of from 3 to 30 °C, preferably from 5 to 20°C.

Preferably the extraction is for a time period of from 5 to 60 minutes, or even from 10 to 45 minutes.

The extraction may be carried out in any suitable contacting equipment, for example a stirred tank.

Preferably the water-to-leaf weight ratio is from 5:1 to 50:1, more preferably from 10:1 to 30:1.

Following extraction the extract is preferably filtered to remove the leaves. The liquor is then preferably centrifuged to remove any coarse material which manages to pass through the filter. Another preferential step is demineralisation of the liquor.

Preferably, the cold-water extract is also demineralised by any suitable process known in the art.

### Nanofiltration step

The nanofiltration is preferably preceded by a microfiltration step as is conventional in the art, in order to protect the nanofilter from coarse particles. Preferably the microfiltration step uses a filter with a pore size of 0.2 micrometres.

The purpose of the nanofiltration step is to enrich the theanine content of the tea solids in the cold-water extract. A cold-water extract will contain tea solids having approximately 6wt% theanine. Thus, concentration to a powder would result in a 6wt% theanine powder.

It is an essential feature of the present invention that the cold-water extract is passed through a nanofilter having a theanine rejection of less than 50%. In this way, the majority of the theanine passes through the filter but the majority of unwanted tea solids remains in the retentate. A single pass through such a filter can double the concentration of theanine in the dry solids.

As the person skilled in the art will understand, nanofilters are characterised in a number of ways according to their intended purpose. It is not physically meaningful to characterise them with an equivalent pore size, as in microfiltration. For the purposes of the present invention, a commercially available nanofilter must be characterised to obtain its theanine rejection percentage. This is carried out at room temperature with a TMP of 5.1 bar and using a theanine solution of concentration 200 to 400 mg/l. For example, the following commercially available filters were characterised and their theanine rejection percentages measured:

**Table 1**

| **Membrane** | **Theanine rejection %** |
|---|---|
| Nitto-Denko NTR 7450 | 7 |
| Trisep NX45 | 35 |
| Dow NF90 | 86 |
| Dow-Filmtec. NF200 | 79 |
| Osmonics DK | 87 |
| Osmonics DL | 88 |

Only the NTR 7450 and the Trisep NX45 are suitable nanofilters from the six nanofilters in table 1.

### Optional concentration step

Usually the enriched extract will need to be concentrated because it normally comprises over 99wt% water. This may be achieved by passing the enriched extract through another nanofilter, however, this time one with a theanine rejection of greater than 80%. In this way, a retentate is provided with most of the theanine but with an order of magnitude less water.

Another way of concentrating is to use reverse osmosis. Such a process will act as a purely concentration step as only water is permitted to pass the filter in such a process.

Because of the thermal instability of theanine, it is preferred that the concentration step does not involve the temperature of the extract exceeding 80 for more than 20 minutes and does not exceed 60 for more than 40 minutes.

The cold water extract can be further concentrated to form a liquid concentrate or a powdered cold water extract. This may be achieved by freeze drying for example. The final concentrate can comprise at least 20 wt%, preferably at least 40 wt%, preferably at least 60 wt%, more preferably at least 80 wt% tea solids.

Preferably the cold-water extract is also treated with polyvinyl pyrolidone to precipitate polyphenolics.

### Optional hot water extraction

When the cold water extraction of the present invention is carried out, the tea leaves are such that they can still be used for the purposes of providing tea extract in a conventional ice tea production process. Therefore there is no waste of tea leaves whilst also obtaining good extraction of the amino acids.

### EXAMPLES

Black tea leaves were infused in water at a temperature of 5°C for a duration of 10 minutes (Cmins = 50). The tea leaves were separated from the liquor which was then centrifuged to remove coarse material, leaving an aqueous tea extract having 0.58wt% dry solids comprising 5.9 wt% theanine upon analysis.

The liquor was then passed through a 0.2 micrometre microfiltration step, to remove any fractions which could damage the nanofilter. The permeate was then passed through a nanofiltration step using a Trisep NX45 filter, resulting in a permeate having 0.065wt% dry solids but comprising 15wt% theanine upon analysis.

The permeate was passed through a reverse osmosis step to remove water, yielding a liquor having approximately 10wt% dry solids, comprising 15wt% theanine. Such a liquor could be further freeze dried to provide a 15wt% theanine powder.

## Claims

1. A process to provide a theanine-rich tea extract comprising the steps of:
(i) performing a cold water extraction of tea plant material using water at a temperature of from 1 to 30°C for a time period of from 1 to 120 minutes to provide a cold-water tea extract;
(ii) passing the extract through a nanofiltration step wherein the nanofilter has a theanine rejection of less than 50% to provide a permeate having tea solids comprising from 8 to 40 wt% theanine.

2. A process according to claim 1, wherein the starting material is fermented tea plant material.

3. A process according to claim 1 or claim, 2, wherein the starting material comprises tea stem.

4. A process according to any preceding claim, wherein the water is at a temperature of from 3 to 30 °C, preferably from 5 to 20°C.

5. A process according to any preceding claim, wherein the cold-water extract is also demineralised.

6. A process according to any preceding claim, wherein the cold water extraction is for a time period of from 5 to 60 minutes.

7. A process according to any preceding claim, wherein a hot water extraction is performed on the tea leaves which have been cold-water extracted using water at a temperature of from 50 to 100°C to provide a hot-water tea extract.

8. A process according to claim 7, wherein the retentate from the nanofiltration step is fed to the hot extraction step.

9. A process according to any preceding claim, wherein the cold-water extract is also treated with polyvinyl pyrolidone to precipitate polyphenolics.

10. A cold-water tea extract comprising tea solids, **characterised in that** the tea solids comprise from 8 to 40 wt% naturally occurring theanine.

11. A cold-water tea extract according to claim 10, comprising more than 98wt% water and less than 2wt% tea solids.

12. A cold-water tea extract according to claim 10, comprising from 40 to 100 wt% tea solids.

13. A cold-water tea extract according to any one of claims 10 to 12, wherein the tea solids comprise from 10 to 25 wt% naturally occurring theanine.

## Patentansprüche

1. Verfahren, um ein theaninreiches Extrakt vorzusehen, umfassend die Schritte:
(i) Durchführen einer Kaltwasserextraktion des Teepflanzenmaterials unter Verwendung von Wasser bei einer Temperatur von 1 bis 30°C für einen Zeitraum von 1 bis 120 Minuten, um ein Kaltwasser-Teeextrakt vorzusehen;
(ii) Passieren des Extrakts durch einen Nanofiltrationsschritt, worin der Nanofilter eine Theanin-Zurückweisung von weniger als 50% hat, um ein Permeat mit Teefeststoffen vorzusehen, umfassend von 8 bis 40 Gew.-% Theanin.

2. Verfahren gemäß Anspruch 1, worin das Ausgangsmaterial fermentiertes Teepflanzenmaterial ist.

3. Verfahren gemäß Anspruch 1 oder Anspruch 2, worin das Ausgangsmaterial Teestängel umfasst.

4. Verfahren gemäß einem vorhergehenden Anspruch, worin das Wasser eine Temperatur von 3 bis 30°C, vorzugsweise von 5 bis 20°C hat.

5. Verfahren gemäß einem vorhergehenden Anspruch, worin das Kaltwasserextrakt ebenfalls entmineralisiert wird.

6. Verfahren gemäß einem vorhergehenden Anspruch, worin die Kaltwasserextraktion für einen Zeitraum von 5 bis 60 Minuten ist.

7. Verfahren gemäß einem vorhergehenden Anspruch, worin eine Heißwasserextraktion an den Teeblättern, welche Kaltwasser extrahiert worden sind, unter Verwendung von Wasser bei einer Temperatur von 50 bis 100°C durchgeführt wird, um ein Heißwasser-Teeextrakt vorzusehen.

8. Verfahren gemäß Anspruch 7, worin das Retentat aus dem Nanofiltrationsschritt zum Heißextraktionsschritt zugeführt wird.

9. Verfahren gemäß einem vorhergehenden Anspruch, worin das Kaltwasserextrakt ebenfalls mit Polyvinylpyrrolidon behandelt wird, um Polyphenole auszufällen.

10. Teefeststoffe umfassendes Kaltwasser-Teeextrakt, **dadurch gekennzeichnet, dass** die Teefeststoffe von 8 bis 40 Gew.-% natürlich vorkommendes Theanin umfassen.

11. Kaltwasser-Teeextrakt gemäß Anspruch 10, welches mehr als 98 Gew.-% Wasser und weniger als 2 Gew.-% Teefeststoffe umfasst.

12. Kaltwasser-Teeextrakt gemäß Anspruch 10, welches von 40 bis 100 Gew.-% Teefeststoffe umfasst.

13. Kaltwasser-Teeextrakt gemäß einem von Ansprüchen 10 bis 12, worin die Teefeststoffe von 10 bis 25 Gew.-% natürlich vorkommendes Theanin umfassen.

## Revendications

1. Procédé de fourniture d'un extrait de thé riche en théanine comprenant les étapes consistant à :
(i) réaliser une extraction à l'eau froide d'une substance végétale de thé en utilisant de l'eau à une température allant de 1 à 30°C pendant une durée allant de 1 à 120 minutes pour fournir un extrait de thé à l'eau froide ;
(ii) soumettre l'extrait à une étape de nanofiltration dans laquelle le nanofiltre a un rejet de théanine de moins de 50 % pour fournir un perméat ayant des particules solides de thé comprenant de 8 à 40 % en poids de théanine.

2. Procédé selon la revendication 1, dans lequel la substance de départ est une substance végétale de thé fermentée.

3. Procédé selon la revendication 1 ou la revendication 2, dans lequel la substance de départ comprend des tiges de thé.

4. Procédé selon l'une quelconque des revendications précédentes, dans lequel l'eau est à une température allant de 3 à 30°C, de préférence de 5 à 20°C.

5. Procédé selon l'une quelconque des revendications précédentes, dans lequel l'extrait à l'eau froide est également déminéralisé.

6. Procédé selon l'une quelconque des revendications précédentes, dans lequel l'extraction à l'eau froide est effectuée pendant une durée allant de 5 à 60 minutes.

7. Procédé selon l'une quelconque des revendications précédentes, dans lequel une extraction à l'eau chaude est réalisée sur les feuilles de thé qui ont été extraites à l'eau froide, en utilisant une eau à une température allant de 50 à 100°C pour fournir un extrait de thé à l'eau chaude.

8. Procédé selon la revendication 7, dans lequel le rétentat de l'étape de nanofiltration est alimenté à l'étape d'extraction à chaud.

9. Procédé selon l'une quelconque des revendications précédentes, dans lequel l'extrait à l'eau froide est également traité avec de la polyvinylpyrrolidone pour précipiter les composés polyphénoliques.

10. Extrait de thé à l'eau froide comprenant des particules solides de thé, **caractérisé en ce que** les particules solides de thé comprennent de 8 à 40 % en poids de théanine naturelle.

11. Extrait de thé à l'eau froide selon la revendication 10, comprenant plus de 98 % en poids d'eau et moins de 2 % en poids de particules solides de thé.

12. Extrait de thé à l'eau froide selon la revendication 10, comprenant de 40 à 100 % en poids de particules solides de thé.

13. Extrait de thé à l'eau froide selon l'une quelconque des revendications 10 à 12, dans lequel les particules solides de thé comprennent de 10 à 25 % en poids de théanine naturelle.
